# EUROPÄISCHE PATENTSCHRIFT

(11) **EP 0 734 719 B1**
(45) Veröffentlichungstag und Bekanntmachung des Hinweises auf die Patenterteilung: **28.03.2001**
(21) Anmeldenummer: 96102029.4
(22) Anmeldetag: 13.02.1996
(51) Int. Cl.: A61K 9/00, A61K 9/14, A61K 9/50, A61K 9/16

(54) **Verfahren und Vorrichtung zur Herstellung von Aerosolen sowie deren Verwendung**
Method and device to manufacture aerosols and their use
Procédé et appareil pour la préparation d'aérosols et leur emploi

(30) Priorität: 03.03.1995 DE 19507410
(43) Veröffentlichungstag der Anmeldung: 02.10.1996
(73) Patentinhaber: GSF-Forschungszentrum für Umwelt und Gesundheit, GmbH, 85764 Oberschleissheim (DE)
(72) Erfinder: Seemann, Stefan, 82467 Garmisch-Partenkirchen (DE); Heyder, Joachim, Prof. Dr., 80802 München (DE); Niessner, Reinhard, Prof. Dr., 78532 Tuttlingen (DE)

(56) Entgegenhaltungen:
- WO-A-89/07929
- AEROSOL SCIENCE AND TECHNOLOGY, Bd. 3, 1984, Seiten 155-166, XP002006466 Y. OTANI; ET AL.: "Growth and Deposition of Saline Droplets Covered with a monolayer of Surfactant"

## Beschreibung

Die Erfindung betrifft ein Verfahren und eine Vorrichtung zur Herstellung von mikroverkapselten Aerosolen, bei denen die Teilchen eines Medikamentaerosols mit mindestens einer Monolage einer hydrophoben Substanz mikroverkapselt sind.

Die derzeitige Anwendung polydisperser Wirkstoffaerosole in der Inhalationstherapie führt häufig zu unkontrollierter und unwirksamer Deposition von Wirkstoffen im Atemtrakt.

Ein Verkapselungsverfahren zur Untersuchung des Depositionsverhaltens von mikroverkapselten Aerosolen wird in Otani, Y. and Wang, C.S., "Growth and deposition of saline droplets covered with a monolayer of surfactant," Aerosol Science and Technology 3, (1984), S. 155 - 166 beschrieben. Als Verkapselungssubstanz wird Hexadecanol verwendet. Zudem wird die dampfförmige Verkapselungssubstanz durch Gleichgewichtseinstellung bei Raumtemperatur bzw. 37° C erhalten und mit dem Aerosol bei gleicher Temperatur vermischt. Deshalb ist für die Absorption des Hexadecanols auf das Aerosol wegen eines fehlenden Temperaturgradienten eine große Mischkammer und damit eine lange Verweilzeit von bis zu 3 Minuten von Aerosol und dampfförmiger Verkapselungssubstanz notwendig.

Aufgabe der Erfindung ist es, bei einem Verfahren der erfindungsgemäßen Art die Verkapselung des Aerosols effektiver zu gestalten, so daß die Erzeugung eines kontinuierlichen mikroverkapselten Aerosolstromes möglich wird und eine Vorrichtung zur Durchführung des Verfahrens zur Verfügung zu stellen.

Gelöst wird diese Aufgabe durch die kennzeichnenden Merkmale der Patentansprüche 1 und 2. Die Unteransprüche beschreiben vorteilhafte Ausgestaltungen der Vorrichtung. Vorteilhaft ist die Verwendung der Aerosole als Therapeutika, oder deren Verwendung zur Wirkstofftestung im Tierversuch.

Das Verfahren ermöglicht einige grundlegende Neuerungen in der Inhalationstherapie.

Die dreidimensionale Verteilung der deponierten Partikel im Atemtrakt kann stark eingeengt werden. Unerwünschte Deposition in nicht erkrankten Lungenbereichen wird somit erheblich reduziert, was die therapeutische Wirkung des Aerosols erhöht und gleichzeitig die unerwünschten Nebenwirkungen vermindert. Dies führt zu geringerem Verbrauch an Wirkstoffen und damit zu niedrigeren Kosten.

Dosimetrie durch Photometrie des mikroverkapselten Aerosols ist möglich. Dies erlaubt die Bestimmung der tatsächlich deponierten Aerosolmenge und damit eine genauere Dosisberechnung.

Durch die Mikroverkapselung ist das hygroskopische Wachstum eines wasserlöslichen Aerosols um ca. 10 Sekunden unterbunden.

Beim vorliegenden Verfahren geschieht die Verkapselung aufgrund des großen Temperaturunterschiedes zwischen Ringspaltmischer und Kondensationsstrecke in weniger als einer Sekunde, was eine Mischkammer überflüssig macht und die Erzeugung eines kontinuierlichen Stromes erlaubt.

Die Menge der gasförmigen Verkapselungssubstanz ist bei Otani, Y. et al. allein vom Volumenfluß der Transportluft abhängig. Beim vorliegenden Verfahren wird die Verkapselungssubstanz erhitzt, dadurch kann die gebildete Dampfmenge der Verkapselungssubstanz zusätzlich über die Heiztemperatur eingestellt werden. Schließlich verwendet Otani, Y. et al. ein Aerosol mit einem Anzahlmedian von 1 µm, was für eine medizinische Anwendung ungeeignet ist. Bei der vorliegenden Erfindung wird ein Aerosol mit einem Anzahlmedian von 1,6 bis 2,1 µm verwendet.

Dies stellt einen optimalen Größenbereich für eine medizinische Anwendung dar.

### Verkapselungsmaterialien

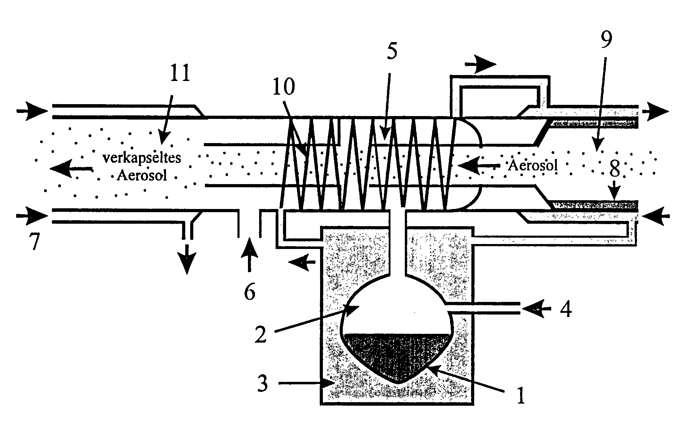

Die Erfindung wird im folgenden anhand eines Ausführungsbeispiels mit Hilfe der Figur näher erläutert.

Dabei zeigt die Figur die schematische Darstellung einer Vorrichtung zur Erzeugung eines mikroverkapselten Aerosolstroms.

Als Aerosolgenerator (hier nicht dargestellt) kann jede Art von Düsen- oder Ultraschallvernebler verwendet werden. Der mit Verkapselungssubstanz 1 gefüllte Spitzkolben 2 taucht in ein thermostatisiertes Wasserbad 3. Dieses ist auf 3° C oberhalb der Schmelztemperatur der Verkapselungssubstanz eingestellt und erhitzt über einen PVC-Schlauch 10 auch den Ringspaltmischer. Durch den Spitzkolben 2 strömt ein vorgewärmter und angefeuchteter Luftstrom 4, der den Dampf von der Oberfläche der Verkapselungssubstanz 1 in den Ringspaltmischer 5 transportiert. Wegen der Thermostatisierung des Spitzkolbens 2 und des Ringspaltmischers 5 durch das gleiche Wasserbad 3, weisen beide Einheiten die gleiche Temperatur auf (3° C oberhalb der Schmelztemperatur der Verkapselungssubstanz). Dies gewährleistet einen verlustfreien Transport der gasförmigen Verkapselungssubstanz, da eine Kondensation aufgrund eines Temperaturgradienten in den Zuleitungen ausgeschlossen ist. Die transportierte Dampfmenge hängt von dem Volumenfluß der Transportluft 4 und stark von der Temperatur der vorgelegten Substanz ab. Eine gute Thermostatisierung ist daher unbedingt erforderlich. Die Befeuchtung der Transportluft geschieht durch zwei Gaswaschflaschen, die mit destillierten Wasser gefüllt sind und in das Wasserbad für die Verkapselungssubstanz 3 eintauchen. Dadurch ist sichergestellt, daß die Transportluft bei der Temperatur des Ringspaltmischers und der Verkapselungssubstanz nahezu wasserdampfgesättigt ist. Bei der Vermischung der Transportluft mit der Aerosolluft kann somit ein Eintrocknen der Tröpfchen vor der Verkapselung ausgeschlossen werden. Die hohe Turbulenz am Ringspalt 5 führt zu einer intensiven und sehr schnellen Vermischung des Aerosols mit der gasförmigen Verkapselungssubstanz. Wie aus der Figur ersichtlich, befindet sich das Aerosol bereits 2 - 3 Sekunden lang im erhitzten Ringspaltmischer 5 bevor es an die Ringspaltdüse gelangt. Das Aerosol wird aber bei Raumtemperatur produziert und, würde somit in dieser Zeit bei der erhöhten Ringspaltmischertemperatur vollständig zu Salzkernen eintrocknen. Um dies zu verhindern muß das Aerosol vorher bei wasserdampfgesättigter-Luft auf die Temperatur des Ringspaltmischers 5 aufgeheizt werden. Zu diesem Zweck ist nach dem Aerosolgenerator eine Konditionierungsstrecke 9 eingebaut. Sie besteht aus einem 35 cm langen Liebig-Kühler, mit einem Innendurchmesser von 2 cm und wird über das Wasserbad für die Verkapselungssubstanz 3 erwärmt und thermostatisiert. Die Innenwände der Konditionierungsstrecke sind mit Filterpapier 8 ausgekleidet, das befeuchtet ist. Je nach Einstellung der Volumenflüße des Aerosolgenerators beträgt die Aufenthaltsdauer des Aerosols in der Konditionierungsstrecke 9 zwischen 6 und 25 Sekunden. Diese Zeit reicht in jedem Fall dazu aus, daß sich das Aerosol mit der erhöhten Temperatur und relativen Luftfeuchtigkeit der Konditionierungsstrecke 9 im Gleichgewicht befindet, wenn es in den Ringspaltmischer 5 gelangt.

Auf den Ringspaltmischer folgt eine Kondensationsstrecke 11, die auf 15° C thermostatisiert ist 7. Grundsätzlich ist ein Temperaturunterschied von mindestens 20° C zwischen Ringspaltmischer 5 und Kondensationsstrecke 11 notwendig um eine quantitative Kondensation des Verkapselungsmaterials auf die Tröpfchenoberfläche des wässerigen Aerosols zu ermöglichen. Damit nicht in störender Weise auch eine Kondensation an den Wänden der Kühlstrecke erfolgt, ist letztere so konstruiert, daß sich ein Mantelluftstrom 6 um den zentralen Aerosolstrom legt, damit dieser mit den gekühlten Glaswänden nicht in Berührung kommen kann. Um eine nahezu gleichmäßige laminare Parallelführung der Strömungslinien zu erreichen und dadurch eine relativ einheitliche Beschichtung zu gewährleisten beträgt der Volumenfluß der Mantelluft ca. 5 % des Volumenflußes des zentral zugeführten Aerosols. Somit können die Tröpfchen definiert beschichtet werden, ohne daß sich dabei ihre Massenund Anzahlverteilung signifikant verändert.

Als Verkapselungsmaterialien eignen sich alle Fettsäuren und Mono- und Dicarbonsäuren mit und ohne Substituenten, die über eine C1 - C18 Kohlenstoffkette als Grundgerüst verfügen. Ebenso alle Alkohole mit und ohne Substituenten mit einer C1- C18 Kohlenstoffkette.

Grundsätzlich kann dieser neu entwickelte Aufbau bei allen bekannten wie auch zukünftigen Medikamentaerosolen eingesetzt werden, die von Pulverinhalatoren, Dosieraerosolen, Düsen- und Ultraschallverneblern erzeugt werden und deren Partikel-Größenverteilungen eine Abhängigkeit von veränderter relativer Luftfeuchte zeigen. In der Tabelle sind einige in der Inhalationstherapie verwendete Wirkstoffe und die dazugehörigen Medikamente aufgelistet, bei denen die Aerosolmikroverkapselung eingesetzt werden kann.

### Bezugszeichenliste:

- 1: Verkapselungssubstanz
- 2: Spitzkolben
- 3: Thermostatisiertes Wasserbad
- 4: Wasserdampfgesättigte Transportluft
- 5: Ringspaltmischer
- 6: Mantelluft
- 7: Thermostatisiertes Kühlwasser
- 8: Filterpapier
- 9: Konditionierungsstrecke
- 10: PVC-Schlauch als Heizschlauch
- 11: Kondensationsstrecke

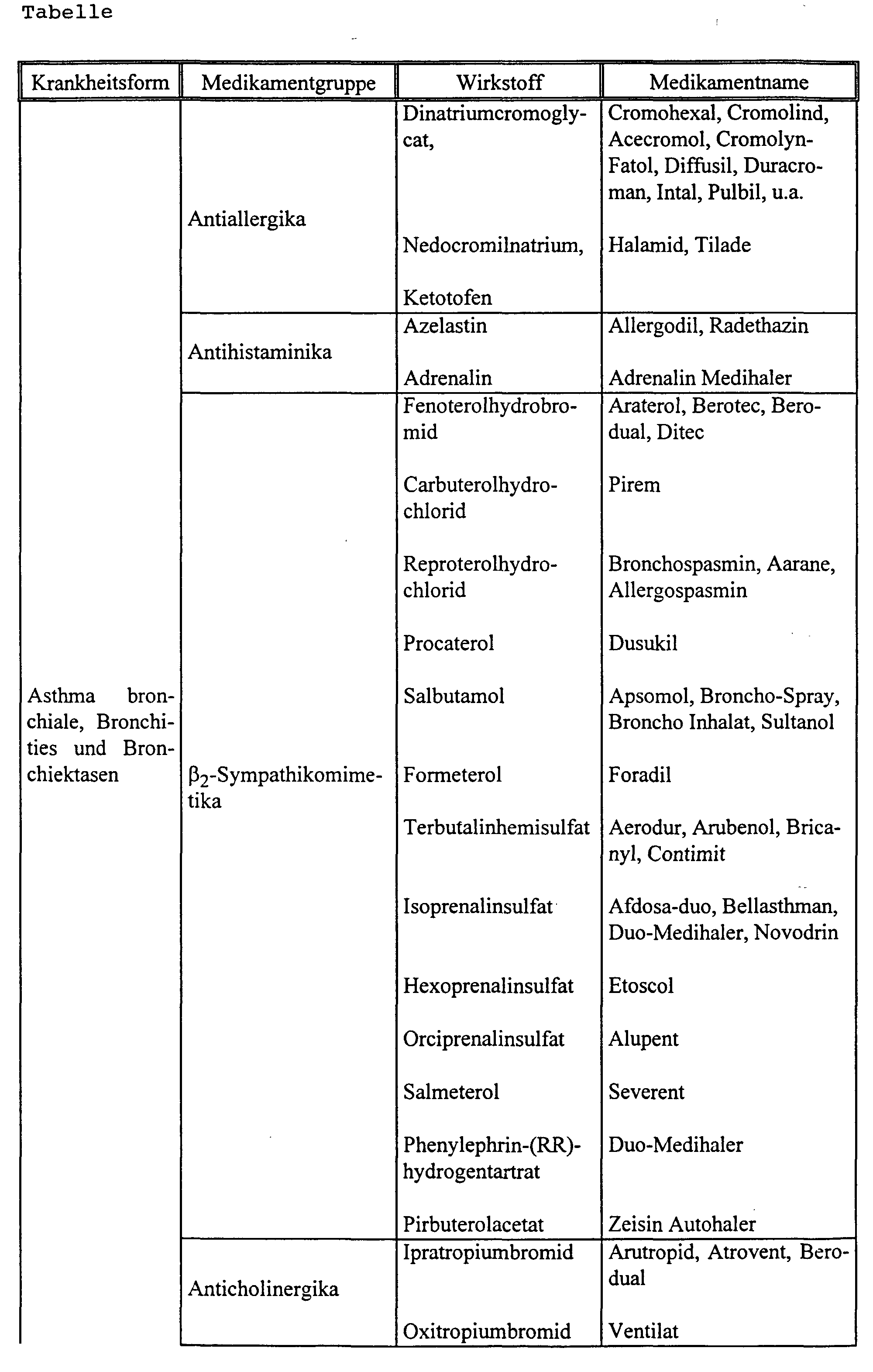

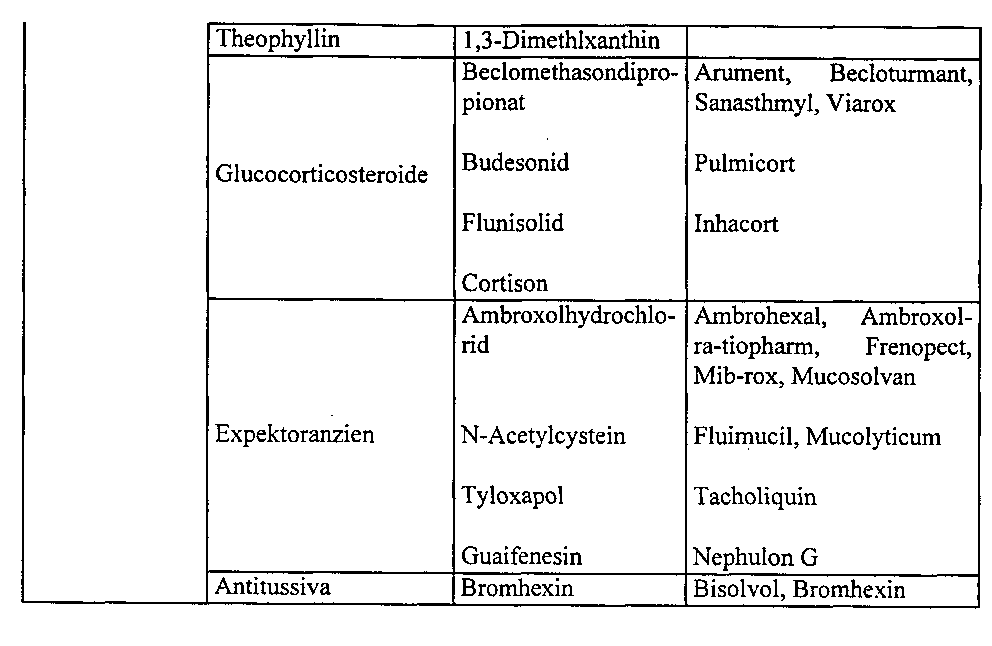

## Patentansprüche

1. Verfahren zur Herstellung von Aerosolen, bei denen die Teilchen eines Medikamentaerosols mit mindestens einer Monolage einer hydrophoben Substanz mikroverkapselt sind, gekennzeichnet durch folgende Verfahrensschritte:
a) Erzeugen eines Medikamentaerosolstromes in gesättigter Wasserdampfatmosphäre bei einer vorwählbaren Temperatur,
b) Erzeugen eines Stromes gasförmiger hydrophober Substanzen in gesättigter Wasserdampfatmosphäre bei der Temperatur des Medikamentaerosols,
c) turbulente Vermischung der beiden Ströme durch Zusammenführen zu einem gerichteten Strom und
d) definierte Abkühlung des zusammengeführten gerichteten Stroms zur Erzeugung der Mikroverkapselung.

2. Vorrichtung zur Herstellung von Aerosolen, bei denen die Teilchen eines Medikamentaerosols mit mindestens einer Monolage einer hydrophoben Substanz mikroverkapselt sind, gekennzeichnet durch:
a) eine Aerosolquelle mit nachfolgender Konditionierungsstrecke (9),
b) Mittel (1, 2, 3, 4) zur Erzeugung eines hydrophoben Substanzstromes,
c) einen Mischer (5) zur turbulenten Vermischung von Aerosol und hydrophobem Substanzstrom und
d) eine kühlbare Kondensationsstrecke (11).

3. Vorrichtung nach Anspruch 2, dadurch gekennzeichnet daß der Mischer (5) ein Ringspaltmischer ist.

4. Vorrichtung nach Anspruch 2 oder 3, gekennzeichnet durch Mittel (3, 10) zur Thermostatisierung der Konditionierungsstrecke (9) und/oder des Mischers (5).

## Claims

1. Method of producing aerosols, wherein the particles of a medicament aerosol are microencapsulated with at least one monolayer of a hydrophobic substance, characterised by the following method steps:
a) producing a medicament aerosol stream in a saturated water vapour atmosphere at a preselectable temperature,
b) producing a stream of gaseous hydrophobic substances in a saturated water vapour atmosphere at the temperature of the medicament aerosol,
c) turbulent mixing of the two streams by bringing them together to form a directed stream; and
d) defined cooling of the directed stream, which has been brought together, to produce the microencapsulation.

2. Apparatus for producing aerosols, wherein the particles of a medicament aerosol are microencapsulated with at least one monolayer of a hydrophobic substance, characterised by:
a) an aerosol source with a subsequent treatment path (9),
b) means (1, 2, 3, 4) for producing a hydrophobic substance stream,
c) a mixer (5) for the turbulent mixing of the aerosol stream and hydrophobic substance stream, and
d) a coolable condensation path (11).

3. Apparatus according to claim 2, characterised in that the mixer (5) is a ring-gap mixer.

4. Apparatus according to claim 2 or 3, characterised by means (3, 10) for thermostatically controlling the treatment path (9) and/or the mixer (5).

## Revendications

1. Procédé de préparation d'aérosols selon lequel les particules d'un médicament à l'état d'aérosol sont micro-encapsulées avec au moins une couche d'une substance hydrophobe,
caractérisé en ce que
a) on génère un flux d'aérosol de médicament dans une atmosphère de vapeur d'eau saturée à une température présélectionnée,
b) on génère un flux de substance hydrophobe à l'état gazeux dans une atmosphère de vapeur d'eau saturée à la température de l'aérosol médicament,
c) on crée un mélange turbulent des deux flux par réunion dans un flux dirigé,
d) on effectue un refroidissement défini des flux réunis en direction pour créer le micro-encapsulage.

2. Dispositif de préparation d'aérosols selon lequel les particules d'un aérosol médicament sont micro-encapsulées avec au moins une substance hydrophobe selon une couche unique,
caractérisé par
a) une source d'aérosol suivie d'un chemin de conditionnement (9),
b) des moyens (1, 2, 3, 4) pour créer un flux de substance hydrophobe,
c) un mélangeur (5) pour réaliser un mélange turbulent d'un flux d'aérosol et de substances hydrophobes et
d) un chemin de condensation (11) susceptible d'être refroidi.

3. Dispositif selon la revendication 2,
caractérisé en ce que
le mélangeur (5) est un mélangeur annulaire.

4. Dispositif selon l'une quelconque des revendications 2 ou 3,
caractérisé par
des moyens (3, 10) pour régler la température de manière thermostatique du chemin de conditionnement (9) et/ou du mélangeur (5).
